# EUROPEAN PATENT SPECIFICATION

(11) **EP 4 663 232 B1**
(45) Date of publication and mention of the grant of the patent: **18.11.2026**
(21) Application number: 25181693.0
(22) Date of filing: 10.06.2025
(51) Int. Cl.: A61N 1/365, A61N 1/37, A61N 1/375, A61N 1/39

(54) **IMPLANTABLE MEDICAL DEVICE FOR ATRIAL SENSING USING AT LEAST ONE VENTRICULAR ELECTRODE**
IMPLANTIERBARE MEDIZINISCHE VORRICHTUNG ZUR ATRIALEN ERFASSUNG UNTER VERWENDUNG MINDESTENS EINER VENTRIKULÄREN ELEKTRODE
DISPOSITIF MÉDICAL IMPLANTABLE DE DÉTECTION AURICULAIRE UTILISANT AU MOINS UNE ÉLECTRODE VENTRICULAIRE

(30) Priority: 12.06.2024 US 202463659018 P; 06.06.2025 US 202519230924
(43) Date of publication of application: 17.12.2025
(60) Divisional application: 26209347.9
(73) Proprietor: Pacesetter, Inc., Sylmar, CA 91342 (US)
(72) Inventor: Sprowls, Mark, Sylmar, CA 91342 (US); Bornzin, Alexander R., Sylmar, CA 91342 (US); Li, Wenwen, Sylmar, CA 91342 (US); Dawoud, Fady, Sylmar, CA 91342 (US)
(74) Representative: Barker Brettell Sweden AB

(56) References cited:
- WO-A1-2023/089415
- US-A1- 2009 306 486
- US-B2- 11 607 550
- US-B2- 11 717 691

## Description

### TECHNICAL FIELD

Embodiments of the present disclosure relate generally to implantable electronic devices for monitoring cardiac activity of a patient and delivering stimulation therapy to the patient's heart.

### BACKGROUND

Some implantable medical devices (IMDs) are designed to monitor cardiac activity in multiple chambers of the heart to determine when it is appropriate to deliver pacing pulses, defibrillation shocks, and/or the like. These IMDs may perform dual chamber sensing to maintain atrioventricular (AV) synchrony between the atrium and the ventricle. Known IMDs that perform dual chamber sensing include a first set of dedicated electrodes for monitoring the right atrium and a second set of dedicated electrodes for monitoring the right ventricle. For example, some known IMDs have multiple leads that extend into the patient's heart. The first set of electrodes for atrial sensing may be disposed on a first lead, and the second set of electrodes for ventricular sensing may be disposed on a second lead.

Other known IMDs use leads that have many electrodes along the length, with at least one of the electrodes located within the right atrium (RA) and at least a second electrode within the right ventricle (RV). Some leads can have at least five discrete electrodes. For example, the electrodes can include a distal tip electrode, an RV ring electrode, an RV coil electrode, two RA ring electrodes, and a second coil electrode in the RA or proximal to the RA. Each electrode is connected to a different discrete electrical signal conductor that extends through the lead to the proximal end of the lead for connecting to a header of the IMD. Leads that have many (e.g., at least five) electrodes may be relatively thick to accommodate the multitude of electrodes and conductors.

Thus, known IMDs that provide dual chamber sensing have multiple leads and/or a single lead that is relatively thick and complex to contain both RV-dedicated electrodes and RA-dedicated electrodes. Implanting multiple leads and/or a thick lead increases the risk of complications, dislodgement, and more complicated extraction procedures relative to implanting a single, thinner lead.

A need remains for an IMD that avoids at least some of the issues with known dual chamber sensing systems that deliver stimulation therapy. For example, a need remains for an IMD that is capable of maintaining AV synchrony without multiple leads and without a large lead. A need remains for an IMD that can perform atrial sensing without dedicated atrial sensing electrodes.

US 2009/306486 A1 discloses an apparatus comprising an implantable cardiac signal sensing circuit, configured to provide a sensed near-field depolarization signal from a ventricle and to provide a sensed a far-field intrinsic atrial signal using a far-field atrial sensing channel, and a controller circuit communicatively coupled to the cardiac signal sensing circuit. The controller circuit includes a P-wave detection module configured to detect an atrial depolarization in the sensed far-field intrinsic atrial signal and a tachyarrhythmia detection module configured to detect an episode of tachyarrhythmia using the sensed near-field depolarization signal and to determine whether the tachyarrhythmia episode is indicative of supraventricular tachycardia (SVT) using the detected atrial depolarization and the sensed near-field depolarization signal.

US 11 717 691 B2 relates to cardiac pacing performed using leadless pacemakers (LPs). An AV delay is determined based on a P-wave duration. When pacing occurs during cardiac cycles starting with intrinsic atrial events, the AV delay is set to the P-wave duration plus a first offset if the P-wave duration is greater than a first threshold duration, and the AV delay is set to the P-wave duration plus a second offset that is greater than the first offset, if the P-wave duration is less than the first threshold duration. When pacing occurs during cardiac cycles starting with paced atrial events, the AV delay is set to the P-wave duration plus a third offset, if the P-wave duration is greater than a second threshold duration, or is set to the P-wave duration plus a fourth offset that is greater than the third offset, if the P-wave duration is less than the second threshold duration.

US 11 607 550 B2 discloses a medical device configured to sense a cardiac electrical signal and determine from the cardiac electrical signal at least one of a maximum peak amplitude of a positive slope of the cardiac electrical signal and a maximum peak time interval from a pacing pulse to the maximum peak amplitude. The device is configured to determine a capture type of the pacing pulse based on at least one or both of the maximum peak amplitude and the maximum peak time interval.

WO 2023/089415 A1 discloses a medical device configured to receive a cardiac signal and determine a morphology matching score from the cardiac signal and a capture detection morphology template that corresponds to a first type of cardiac pacing capture that includes capture of at least a first portion of the His-Purkinje system. The device is configured to detect a second type of cardiac pacing capture in response to the morphology matching score being less than the first match threshold. The second type of cardiac pacing capture is different than the first type of cardiac pacing capture and may include capture of the ventricular myocardium and/or a second portion of the His-Purkinje system different than the first portion.

### SUMMARY

The invention is defined in the appended claims. Aspects, embodiments and examples disclosed herein which do not fall within the scope of the appended claims do not form part of the invention, and are merely provided for illustrative purposes.

In accordance with embodiments herein, an IMD is provided that includes a housing, a right ventricle (RV) near field (NF) electrode, a far field (FF) electrode, and sensing circuitry. The housing contains one or more processors. The RV NF electrode is electrically connected to the one or more processors and configured to be located within the RV of a heart of a patient and either in contact with ventricular myocardial tissue of the heart or within a threshold proximity of the ventricular myocardial tissue. The FF electrode is configured to be positioned beyond the threshold proximity of the ventricular myocardial tissue. The IM comprises a lead having the RV NF and FF electrodes. The sensing circuitry is coupled to the RV NF electrode and the FF electrode and configured to define an atrial sensing vector to collect atrial sensing data associated with atrial cardiac activity. The one or more processors are configured to receive the atrial sensing data and perform VDD mode pacing on the heart based on the atrial sensing data.

In an embodiment, the implantable medical device includes a third electrode. The sensing circuitry is coupled to the third electrode and configured to define a ventricular sensing vector between the third electrode and the RV NF electrode. The ventricular sensing vector is configured to collect ventricular sensing data.

In an embodiment, the RV NF electrode is one of an RV tip electrode or an RV ring electrode.

In an embodiment, the sensing circuitry is configured to establish a conductive pathway from the RV NF electrode to both an RV terminal and right atrium terminal on or within the housing.

In an embodiment, the implantable medical device includes a lead having the RV NF and FF electrodes. The RV NF electrode may be located proximate to a distal end of the lead, and the FF electrode may be spaced apart from the RV NF electrode by at least 1 cm.

In an embodiment, the FF electrode is a coil electrode configured to be located near or partially within a right atrium of the heart.

In an embodiment, the lead is the only lead of the implantable medical device.

In an embodiment, the RV NF electrode is electrically connected to a first conductor that extends along a length of the lead to a proximal end of the lead. The first conductor is electrically connected to both a first RV terminal of the housing and a first right atrium (RA) terminal of the housing so that signals collected by the RV NF electrode are conveyed to both the first RV terminal and the first RA terminal.

In an embodiment, the FF electrode is a coil electrode and is electrically connected to a second conductor that extends along a length of the lead to the proximal end of the lead. The second conductor is electrically connected to both a second RV terminal of the housing and a second RA terminal of the housing so that signals collected by the coil electrode are conveyed to both the second RV terminal and the second RA terminal.

In an embodiment, the implantable medical device is free of any atrial electrodes.

In an embodiment, the implantable medical device is an implantable cardioverter-defibrillation (ICD) device or a cardiac resynchronization therapy-defibrillation (CRT-D) device.

In an embodiment, the implantable medical device (IMD) is a leadless IMD. The RV NF electrode is provided at a distal end of the housing and is configured to be located adjacent to ventricular myocardial tissue. The FF electrode is provided at a proximal end of the housing and configured to be located within a cavity of the RV.

In an embodiment, the one or more processors are configured to analyze the atrial sensing data collected via the atrial sensing vector to detect P-waves over time and determine an atrial rate based on the P-waves that are detected.

In an embodiment, the one or more processors are configured to detect the P-waves by (i) defining an atrial sensitivity upper limit and an atrial sensitivity lower limit; (ii) monitoring an absolute signal amplitude of the atrial sensing data over time; (iii) triggering R/T-wave refractory periods responsive to the absolute signal amplitude exceeding the atrial sensitivity upper limit; and (iv) identifying the P-waves at times that the absolute signal amplitude is between the atrial sensitivity upper and lower limits and is outside of the R/T-wave refractory periods.

In an embodiment, the one or more processors are configured to diagnose atrial tachyarrhythmia based on the atrial rate.

In an embodiment, the atrial sensing vector is a first atrial sensing vector of multiple different candidate atrial sensing vectors defined by RV NF electrodes and FF electrodes of the implantable medical device. The one or more processors are configured to select the first atrial sensing vector for collecting the atrial sensing data during a sinus rhythm mode, and select a second atrial sensing vector of the candidate atrial sensing vectors for collecting atrial sensing data during a ventricular pacing mode.

### BRIEF DESCRIPTION OF THE DRAWINGS

Figure 1 illustrates a schematic cutaway view of a heart relative to an IMD.
Figure 2 is a schematic block diagram of an IMD formed in accordance with at least one embodiment.
Figure 3 illustrates an IMD implanted in a patient's heart according to a first example application.
Figure 4 illustrates the IMD shown in Figure 3 implanted in a patient's heart according to a second example application.
Figure 5 illustrates an IMD implanted in a patient's heart according to a third example application.
Figure 6 illustrates an IMD implanted in a patient's heart according to a fourth example application.
Figure 7 is a flow chart of a method for selecting the atrial sensing vector for a sinus rhythm cardiac mode.
Figure 8 is a flow chart of a method for selecting the atrial sensing vector for the pacing mode of operation.
Figure 9 is a flow chart of a method of detecting P-waves in cardiac data.
Figure 10 is a graph depicting atrial signal amplitude over time.
Figure 11 illustrates a block diagram of an exemplary IMD that is configured to be implanted into the patient in accordance with one or more embodiments herein.

### DETAILED DESCRIPTION

It will be readily understood that the components of the embodiments as generally described and illustrated in the figures herein, may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The term "sensing vector" shall mean a path extending between two or more physical, actual electrodes that operate as sensing sites.

The term "near field" shall mean within a chamber of interest and in contact with myocardial tissue of the chamber of interest or within a threshold proximity of the myocardial tissue of the chamber of interest. The term "near field electrode" shall mean an electrode that is near field and configured to sense activity of the chamber of interest and/or deliver electrical stimulation to the myocardial tissue of the chamber of interest. The terms "right ventricular near field electrode" and "RV near field electrode" shall mean an electrode configured to be located within the RV of the heart and either in contact with ventricular myocardial tissue of the heart or within a threshold proximity of the ventricular myocardial tissue. Example RV near field electrodes can include an RV tip electrode and an RV ring electrode.

The term "threshold proximity" shall mean a designated distance value from myocardial tissue of a patient's heart. Example threshold proximities may include 5 mm, 8 mm, 10 mm, 20 mm, or the like. The threshold proximity may be selected based on application-specific parameters, patient-specific parameters, clinician preferences, and/or the like. Electrodes that are within the threshold proximity of the myocardial tissue can effectively sense activity of the myocardial tissue and/or deliver electrical stimulation to the myocardial tissue.

The term "far field" shall refer to both (i) outside of a chamber of interest and (ii) at least partially within the chamber of interest but not in contact with myocardial tissue of the chamber of interest and beyond a threshold proximity of the myocardial tissue of the chamber of interest. The term "far field" may include objects that are implanted to be located in the blood pool of a chamber of the heart, objects that are implanted outside of a chamber of the heart, and objects that are implanted in the patient at locations remote from the heart. The term "far field electrode" shall mean an electrode that is not in contact with the myocardial tissue or within the threshold proximity of the myocardial tissue. In an example, the chamber of interest may be the RV. In this example, the far field electrode is not in contact with the ventricular myocardial tissue and is not within the threshold proximity of the ventricular myocardial tissue. The far field electrode according to the example may be located in the blood pool of the RV, in the RA, in the SVC, or implanted in the patient at a location remote from the heart.

The terms "cardiac activity signal", "cardiac activity signals", "CA signal" and "CA signals" (collectively "CA signals") are used interchangeably throughout to refer to measured signals indicative of cardiac activity by a region or chamber of interest. Cardiac activity refers to electrophysiological events (e.g., depolarization and polarization) of the cardiac regions or chambers of the heart over time. The cardiac activity is regulated by nodal tissues and produces a cardiac output. For example, the CA signals may be indicative of impedance, electrical or mechanical activity by one or more chambers (e.g., left or right ventricle, left or right atrium) of the heart and/or by a local region within the heart (e.g., impedance, electrical or mechanical activity at the AV node, along the septal wall, within the left or right bundle branch, within the purkinje fibers). The cardiac activity may be normal/healthy or abnormal/arrhythmic. An example of CA signals includes EGM signals. Electrical based CA signals refer to an analog or digital electrical signal recorded by two or more electrodes, where the electrical signals are indicative of cardiac activity. Heart sound (HS) based CA signals refer to signals output by a heart sound sensor such as an accelerometer, where the HS based CA signals are indicative of one or more of the S1, S2, S3 and/or S4 heart sounds. Impedance based CA signals refer to impedance measurements recorded along an impedance vector between two or more electrodes, where the impedance measurements are indicative of cardiac activity. The term "atrial cardiac activity" refers to cardiac activity of an atrial chamber, such as the right atrium.

The term "sensed cardiac events" refers to electrical events or features in a cardiac cycle including atrial depolarization (P-waves), ventricular depolarization (R-waves or QRS complexes), and ventricular repolarization (T-waves).

The term "defibrillation shock" refers to defibrillation stimulus delivered at an energy level sufficient to terminate a defibrillation episode in a heart, wherein the energy level is defined in Joules to be 15 J or more and/or the energy level is defined in terms of voltage to be 100 V or more. The pacing pulses are delivered at a lower energy level than the defibrillation shocks.

The terms "normal" and "sinus" are used to refer to events, features, and characteristics of, or appropriate to, a heart's healthy or normal functioning.

The term "free of any atrial electrodes" shall mean that the IMD, or leads connected to the IMD, do not have any electrode(s) entirely within an atrial chamber (e.g., the RA). For the avoidance of doubt, the phrase "free of any atrial electrodes" does not exclude a ventricular lead having a coil electrode located in a mid-section of the lead body that is partly in the RA and partially in the RV. The primary functions of atrial electrodes are to sense atrial activity and/or to deliver therapy to the atrial myocardium. The RV tip, RV ring, and coil electrodes described herein are not atrial electrodes because these electrodes are not designed to be implanted within an atrial chamber.

The methods described herein may employ structures or aspects of various embodiments (e.g., systems and/or devices) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that, other methods may be used, in accordance with an embodiment herein. Further, wherein indicated, the methods may be fully or partially implemented by one or more processors of one or more devices or systems. While the operations of some methods may be described as performed by the processor(s) of one device, additionally, some or all of such operations may be performed by the processor(s) of another device described herein.

Embodiments may be implemented in connection with one or more implantable medical devices (IMDs). Non-limiting examples of IMDs include neurostimulator devices, implantable monitoring and/or therapy devices, catheters, and/or alternative implantable medical devices. For example, the IMD may represent a cardiac monitoring device, pacemaker, cardioverter, cardiac rhythm management device, defibrillator, neurostimulator, leadless monitoring device, leadless pacemaker and the like. For example, the IMD may include one or more structural and/or functional aspects of the device(s) described in U.S. Patent 9,333,351 "Neurostimulation Method And System To Treat Apnea" and U.S. Patent 9,044,610 "System And Methods For Providing A Distributed Virtual Stimulation Cathode For Use With An Implantable Neurostimulation System."

In another example, the IMD may be a leadless IMD, such as a leadless pacemaker. The leadless IMD may include one or more structural and/or functional aspects of the device(s) described in U.S. Patent 9,216,285 "Leadless Implantable Medical Device Having Removable And Fixed Components" and U.S. Patent 8,831,747 "Leadless Neurostimulation Device And Method Including The Same." Additionally or alternatively, the IMD 180 may include one or more structural and/or functional aspects of the device(s) described in U.S. Patent 8,391,980 "Method And System For Identifying A Potential Lead Failure In An Implantable Medical Device" and U.S. Patent 9,232,485 "System And Method For Selectively Communicating With An Implantable Medical Device." Additionally or alternatively, the IMD may be a leadless cardiac monitor (ICM) that includes one or more structural and/or functional aspects of the device(s) described in U.S. Patent 9,949,660, entitled, "Method And System To Discriminate Rhythm Patterns In Cardiac Activity."

Embodiments set forth herein include IMDs configured to monitor cardiac activity of a patient. The disclosure also include methods performed by the IMDs to monitor cardiac activity of a patient. The disclosure further includes a method of providing stimulation therapy to the patient's heart via the IMD. In particular embodiments, the IMD does not use a dedicated right atrial (RA) lead or even dedicated RA electrodes to sense atrial activity. In an example, the IMD may not include an RA lead or dedicated RA electrodes.

The IMD may provide dual chamber (e.g., atrial and ventricular) sensing and ventricular pacing for maintaining AV synchrony. The IMD may monitor atrial activity (e.g., depolarization) using a bipolar atrial sensing vector that lacks dedicated atrial electrodes. The electrodes of the bipolar atrial sensing vector may be located remote from the atrium. For example, at least one of the electrodes in the atrial sensing vector may be located within the right ventricle (RV). A tip electrode at the distal end of a lead can define one electrode of the atrial sensing pair, even if the tip electrode is at the apex of the right ventricle and/or embedded in the septum wall of the right ventricle.

In an example, the IMD may be used to perform cardiac therapy by monitoring the cardiac activity of the heart and selectively delivering pacing pulses and defibrillation shocks when appropriate. For example, the IMD may include a single lead. The lead have as few as two or three electrodes. The three electrodes may include an RV tip electrode, an RV ring electrode, and a coil electrode. In an example with only two electrodes, the IMD may omit the RV ring electrode or the RV tip electrode. Depending on a programmed configuration, the RV tip electrode or the RV coil electrode may define the cathode in a ventricular pacing vector for delivering pacing pulses to the myocardium. The RV tip or ring electrode may define one of the electrodes in both the atrial sensing vector as well as a ventricular sensing vector, although the atrial sensing vector is different from the ventricular sensing vector. For example, the RV tip electrode or RV ring electrode may be used to sense both atrial cardiac activity and ventricular cardiac activity. The coil electrode may deliver defibrillation shocks to the heart. The lead with three electrodes may have a substantially smaller diameter than a lead with five, six, or more electrodes for monitoring both RA and RV chambers. The embodiments described herein can be implemented with various different types of defibrillation leads that have different numbers, types, and/or arrangements of electrodes. In another example, the IMD described herein may be leadless, and the electrodes may be located along the length of the housing.

In an example, the IMD is an implantable cardioverter-defibrillator (ICD) system and/or a cardiac resynchronization therapy defibrillator (CRT-D) system. The IMD may be capable of functional VDD mode pacing, meaning that the IMD can provide ventricular pacing and dual chamber (RA and RV) sensing. The ICD/CRT-D system described herein may be capable of maintaining AV synchrony without using any dedicated RA electrodes.

In examples described herein, the IMD performs methods to achieve VDD mode pacing for AV synchrony that involve sensing atrial activity using an RV near field electrode. For example, the IMD may sense both atrial activity and ventricular activity without using any dedicated atrial electrodes. For example, the atrial sensing vector is defined by an RV near field electrode and a far field electrode in the form of a coil electrode or a can electrode. The methods described herein include a method for P-wave detection, a method for detecting atrial tachyarrhythmia, and methods to select the atrial sensing vector during different cardiac modes. For example, the algorithms are designed to allow the IMD to maintain accurate sensing for atrial activity by, in part, adjusting atrial sensitivity parameters based on the atrial sensing vector. One or more of the algorithms may allow the IMD to maintain accurate sensing by avoiding or at least reducing P-wave over-sensing.

The IMD and methods described herein provide several beneficial technical effects. For example, a first technical effect of using an RV near field electrode as part of the atrial sensing bipole is that the atrial signal amplitude may be relatively high, which is beneficial to distinguish the sensed atrial activity from noise. The atrial signal amplitude via the atrial sensing vector may be relatively high because the RV near field electrode may not collect any far-field data. For example, the RV near field electrode only monitors signals within the RV. The far field electrode (e.g., can or coil electrode) may collect signals from the RA. The signal amplitude across the atrial sensing pair of electrodes is the difference in the electrode amplitudes. The atrial signal amplitude of the atrial sensing bipole may be relatively high because the atrial signal amplitude of the far field electrode is significantly greater than the signal amplitude of the RV near field electrode. Another technical effect of using the RV near field electrode as part of the atrial sensing bipole may be that the (rectified) P-wave amplitude recorded on the atrial sensing channel can be relatively high (e.g., maximized) using only one far field electrode. A third technical effect of using the RV near field electrode as part of the atrial sensing bipole may be that the R and T-wave durations recorded on the atrial sensing channel may be relatively short. The short R/T wave durations allow for accurately distinguishing P-waves from R and T-waves. P-wave discrimination and detection is used by the IMD for diagnosing atrial arrhythmias, such as atrial tachyarrhythmia.

The IMDs described herein deliver a particular treatment for the medical condition of atrial tachyarrhythmia, ventricular arrhythmia (VA) (e.g., VA episode(s)), and/or the like. The treatment may be selected from a collection of therapies stored in a memory device. The treatment may be selected based on the current posture of the patient and/or the patient's instant medical condition (e.g., heart rate and/or hemodynamic stability). The IMD may deliver the particular treatment which transforms the patient's heart from an arrhythmia state to a normal sinus rhythm state. The IMD may continuously monitor a patient's cardiac signals and provide shock therapy, pacing, etc. as needed to maintain the function of the heart and prevent death and/or unnecessary treatment. The IMD may adjust the particular treatment as the patient's condition changes.

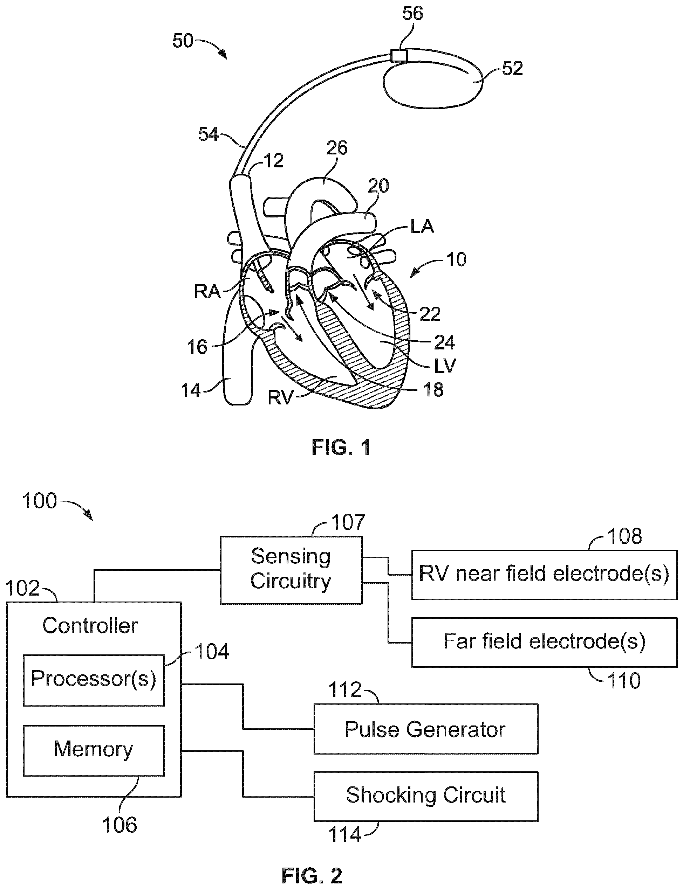

Figure 1 illustrates a schematic cutaway view of a heart 10 relative to an IMD 50. The IMD 50 includes a lead 54 that may be delivered to the heart 10 during an implant procedure. The heart 10 has several chambers including the RA, the RV, a left atrium (LA), and a left ventricle (LV). During normal operation (e.g., sinus rhythm) of the heart 10, deoxygenated blood from the body is returned to the RA from a superior vena cava 12 and an inferior vena cava 14 of the heart 10. The RA pumps the blood through an atrioventricular or tricuspid valve 16 of the heart 10 to the RV, which then pumps the blood through the pulmonary valve 18 and the pulmonary artery 20 to the lungs for reoxygenation and removal of carbon dioxide. The newly oxygenated blood from the lungs is transported to the LA, which pumps the blood through the mitral valve 22 to the LV. The LV pumps the blood through the aortic valve 24 and the aorta 26 throughout the body.

The IMD 50 includes a housing 52 that is operably coupled to the lead 54 through a lead adaptor 56. A proximal end of the lead 54 may be coupled to the pulse generator 52 via the lead adaptor 56 after the lead 54 is delivered to the implant site. The lead 54 may enter the vascular system through one of several possible vascular access sites. For example, the lead 54 may enter through the femoral artery/vein. The lead 54 may extend through the superior vena cava 12 to the right atrium RA. The distal end of the lead 54 may be advanced through the tricuspid valve 16 into the RV.

The IMD 50 may be a dual-chamber sensing device that is capable of providing stimulation therapy to maintain AV synchrony and treat arrhythmias. The stimulation therapy may include cardioversion, defibrillation, and pacing stimulation. The IMD 50 may be controlled to sense atrial and ventricular waveforms of interest, discriminate between two or more ventricular waveforms of interest, deliver stimulus pulses or shocks, and inhibit application of a stimulation pulse to a heart based on the discrimination between the waveforms of interest and the like. The IMD 50 may be an ICD, a CRT-D, an ICD coupled with a pacemaker, and/or the like.

Although not shown, the IMD 50 may wirelessly communicate with an external device. The external device may be used by a physician or other technician to select and/or modify therapy parameters to be implemented by the IMD 50.

Figure 2 is a schematic block diagram of an IMD 100 according to embodiments described herein. The IMD 100 includes a controller 102 that has one or more processors 104. The one or more processors 104 represent hardware circuitry, such as one or more microprocessors, integrated circuits, microcontrollers, field programmable gate arrays, etc.). The controller 102 may include at least one tangible and non-transitory computer-readable storage medium (e.g., data storage device), referred to herein as memory 106. The one or more processors 104 perform some or all of the operations and methods of the IMD described herein. The memory 106 may store program instructions (e.g., software) that are executed by the one or more processors 104 to perform the operations of the IMD described herein. For example, the program instructions stored in the memory 106 may be executable by the one or more processors 104 to analyze atrial sensing data collected via an atrial sensing vector that includes an RV near field electrode 108.

The IMD 100 includes multiple electrodes for defining sensing vectors and stimulation vectors (e.g., for pacing and/or defibrillation). The electrodes include one or more RV near field electrodes 108 and one or more far field electrodes 110. The IMD 100 may include sensing circuitry 107 coupled to the RV near field electrode(s) 108 and the far field electrode(s) 110. The sensing circuitry 107 may be electrically connected to the electrodes 108, 110 via conductive elements such as wires, circuit traces, electrical connectors, and/or the like. The sensing circuitry 107 may define an atrial sensing vector to collect atrial sensing data associated with atrial cardiac activity. The sensing circuitry 107 may be configurable (e.g., programmable) by the controller 102 to define or select the atrial sensing vector from multiple different candidate combinations of electrodes 108, 110. For example, the sensing circuitry 107 may be coupled to more than two electrodes 108, 110. The controller 102 may program the sensing circuitry 107 to define bipolar atrial sensing vector composed of two of the electrodes 108, 110. In an example, the sensing circuitry 107 may be programmed to define different atrial sensing vectors (e.g., to use different combinations of the electrodes 108, 110 to collect atrial sensing data) for different time periods and/or modes of cardiac function. Example modes of cardiac function can include normal sinus rhythm, a ventricular pacing mode associated with delivery of a ventricular pacing pulse, and/or the like.

The IMD 100 may also include circuitry and hardware for delivering stimulation therapy via electrodes. For example, the IMD 100 may include a pulse generator 112 for generating pacing pulses and a shocking circuit 114 for generating defibrillation shocks. The IMD 100 may include additional components than the components shown in Figure 2.

In an example, the components of the IMD 100 shown in Figure 2 may be integrated on the IMD 50 shown in Figure 1. For example, the controller 102, sensing circuitry 107, pulse generator 112, and shocking circuit 114 may be disposed within the housing 52. The RV near field electrode(s) 108 may be located on the lead 54. The far field electrode(s) 110 may be located on the lead 54 and/or on the housing 52. For example, the housing 52 may include or define a can electrode that represents a far field electrode 110. The housing 52 may be referred to as a "can", "case" or "case electrode", and may be programmably selected to act as a return electrode (e.g., anode) in an electrode pair.

The IMD 100 may perform atrial sensing using a bipolar atrial sensing vector defined by the sensing circuitry 107. The bipolar atrial sensing vector may include one RV near field electrode 108 and one far field electrode 110. The RV near field electrode 108 refers to an electrode configured to be located within the RV of the heart. In the implanted location, the RV near field electrode is either in contact with ventricular myocardial tissue of the heart or within a threshold proximity of the ventricular myocardial tissue. The threshold proximity may be a value selected based on application-specific parameters. For example, the threshold proximity may be 5 mm, 8 mm, 10 mm, 20 mm or the like. Example RV near field electrodes can include an RV tip electrode and an RV ring electrode.

The far field electrode 110 refers to an electrode that is not in contact with the ventricular myocardial tissue or within the threshold proximity of the ventricular myocardial tissue. For example, the far field electrode 110 is beyond the threshold proximity of the ventricular myocardial tissue. The far field electrode 110 may be in the blood pool of a chamber of the heart, such as the blood pool in the RA or the RV. In another example, the far field electrode may be located in the SVC. In yet another example, the far field electrode may be implanted in the patient at a location remote from the heart. The far field electrode 110 is spaced apart from the RV near field electrode 108.

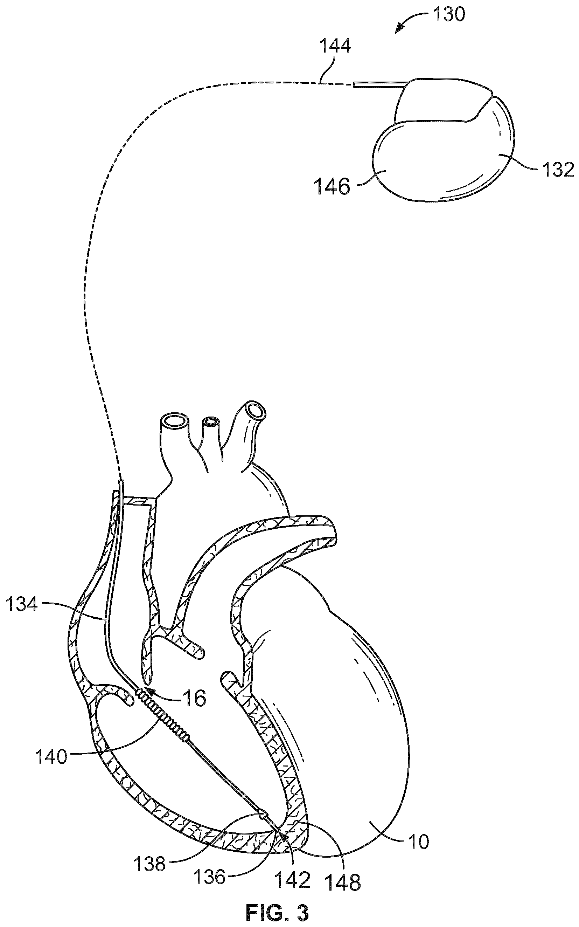

Figure 3 illustrates an IMD 130 implanted in a patient's heart 10 according to a first example application. The IMD 130 may represent the IMD 100 shown in Figure 2. The IMD 130 includes a housing 132 and a lead 134 extending from the housing 132 into the heart 10. The lead 134 extends through the RA into the RV. The lead 134 has multiple electrodes. In the illustrated example, the lead 134 includes an RV tip electrode 136, an RV ring electrode 138, and a coil electrode 140. The coil electrode 140 is designed to deliver defibrillation shocks to the heart. The coil electrode 140 may be referred to as an RV coil electrode 140 because a majority of the coil is within the RV. Optionally, a portion of the RV coil electrode 140 may be disposed within the RA. The RV coil electrode 140 may be spaced apart from the RV NF electrode by at least 1 cm. For example, the RV coil electrode 140 may be located distally (e.g., at least 1 cm from the distal end 142 of the lead 134) or along a midsection of the lead 134. The RV coil electrode 140 may be positioned near (but outside of) or partially within the right atrium of the heart. When implanted, the RV coil electrode 140 may be located proximate to, distal to, or crossing the tricuspid valve 16 of the patient that separates the RV from the RA. For example, the RV coil electrode 140 may extend into and/or through the tricuspid valve 16. The RV tip electrode 136 is located at the distal end 142 of the lead 134. The RV ring electrode 138 is located along the length of the lead 134 between the RV tip electrode 136 and the RV coil electrode 140. The electrodes 136, 138, 140 are electrically connected to electronic circuitry (e.g., the controller 102) within the housing 132 via respective conductors 144 that extend along the length of the lead 134. In the illustrated application, the lead 134 is implanted so that the distal end 142 and the RV tip electrode 136 are located at the right ventricular apex 148.

In an embodiment, the electrodes 136, 138, 140 on the lead 134, and optionally a can electrode 146 of the housing 132, are used to provide dual chamber sensing in both the RA and RV. The IMD 130 uses an atrial sensing vector to monitor atrial activity. The atrial sensing vector may be a bipolar vector defined by an RV near field electrode 108 and a far field electrode 110. The RV tip electrode 136 or the RV ring electrode 138 may be selected as the RV near field electrode 108 in the atrial sensing vector. The far field electrode 110 may be defined by the RV coil electrode 140 or the can electrode 146 (e.g., the housing 132).

The IMD 130 uses a ventricular sensing vector to monitor ventricular activity. The ventricular sensing vector may be an electrode pair including two of the electrodes 136, 138, 140, and 146. For example, a first electrode of the ventricular sensing vector may be defined by the RV tip electrode 136 or the RV ring electrode 138, and a second electrode of the ventricular sensing vector may be defined by the RV coil electrode 140 or the can electrode 146. In an example, the ventricular sensing vector may have one electrode in common with the atrial sensing vector. For example, one electrode may be used in both the atrial sensing vector and the ventricular sensing vector. That electrode may be the RV tip electrode 136 or the RV ring electrode 138. In another example, the ventricular sensing vector may have two electrodes in common with the atrial sensing vector, so that two electrodes are use in both the atrial and ventricular sensing vectors. The same electrode pair (e.g., RV tip electrode 136 and RV coil electrode 140) may be used for both atrial and ventricular sensing.

Furthermore, the IMD 130 may use two of the electrodes 136, 138, 140, and/or 146 to define at least one ventricular stimulation vector for delivering pacing pulses and/or defibrillation shocks to the ventricular myocardial tissue when appropriate. In an example, a ventricular stimulation vector may have one electrode in common with the atrial sensing vector and/or one electrode in common with the ventricular sensing vector.

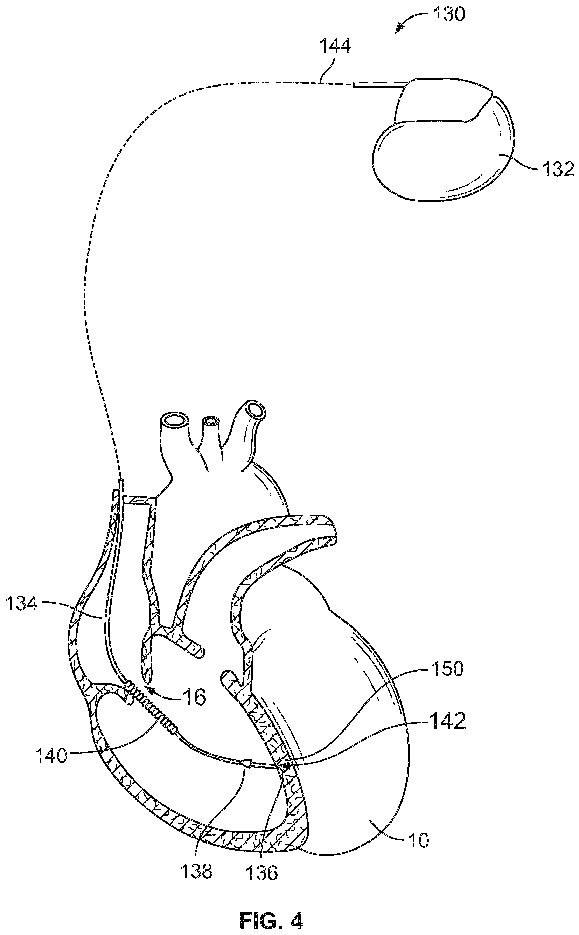

Figure 4 illustrates the IMD 130 shown in Figure 3 implanted in a patient's heart 10 according to a second example application. In Figure 4, the lead 134 is implanted so that the distal end 142 and the RV tip electrode 136 are located at the septal wall 150 between the right and left ventricles. The RV tip electrode 136 may be embedded in the septal wall 150 at or proximate to the left bundle branch (LBB). The RV tip electrode 136 may be used to provide LBB area pacing. The RV coil electrode 140 may be located near or partially within the right atrium of the heart, similar to the application shown in Figure 3. For example, a portion of the RV coil electrode 140 may extend in or through the tricuspid valve 16 between the RA and the RV.

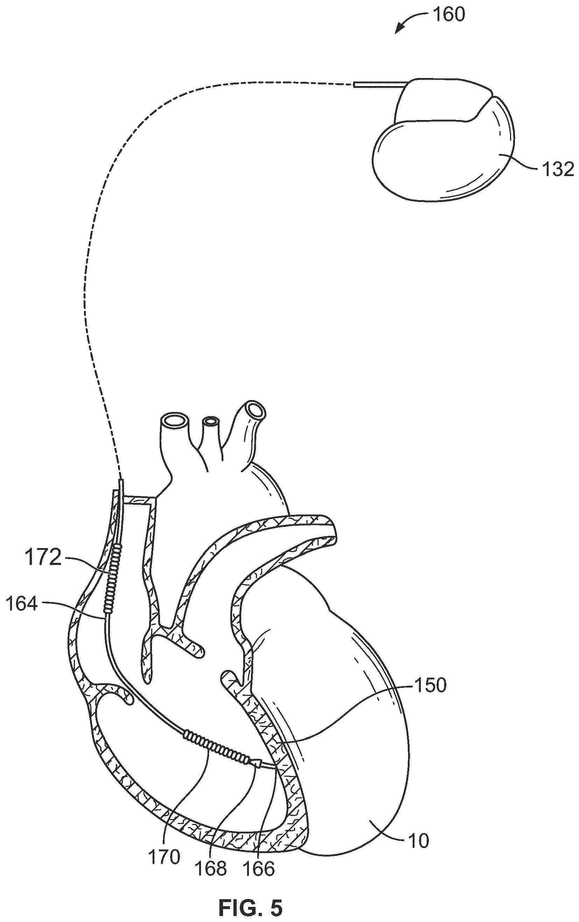

Figure 5 illustrates an IMD 160 implanted in a patient's heart according to a third example application. The IMD 160 may represent the IMD 100 shown in Figure 2. The IMD 160 only differs from the IMD 130 shown in Figures 3 and 4 in the lead that is used. The IMD 160 includes a housing 162 and a lead 164 extending from the housing 162 into the heart 10. The lead 164 extends through the RA into the RV. The lead 164 has multiple electrodes including an RV tip electrode 166, an RV ring electrode 168, an RV coil electrode 170, and a superior vena cava (SVC) coil electrode 172. The RV tip electrode 166 is shown in contact with the septal wall 150 in Figure 5, but may be located at the ventricular apex or another location in the RV in other applications.

The RV tip electrode 166 and the RV ring electrode 168 may represent the RV near field electrodes 108 shown in Figure 2. The RV coil electrode 170, the SVC coil electrode 172, and the housing 162 (i.e., can electrode) may represent the far field electrodes 110. For example, the bipolar atrial sensing vector may be defined by one of the RV tip or ring electrodes 166, 168 and one of the RV coil electrode 170, the SVC coil electrode 172, or the housing 162.

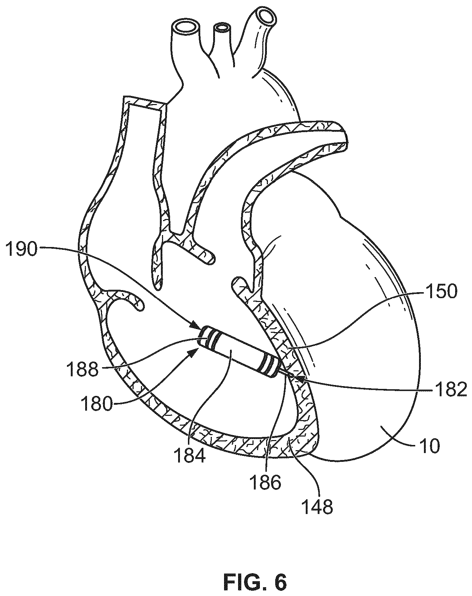

Figure 6 illustrates an IMD 180 implanted in a patient's heart according to a fourth example application. The IMD 180 may represent the IMD 100 shown in Figure 2. The IMD 180 is a leadless device, such as a leadless pacemaker. The IMD 180 is implanted in the RV.

In the illustrated application, a distal end 182 of the IMD 180 is secured to the septal wall 150. In another application, the distal end 182 may be secured to the myocardial tissue at the apex 148. The IMD 180 has a housing 184 that contains electronic circuitry such as the controller 102, the pulse generator 112, and/or the shocking circuit 114. The IMD 180 includes multiple electrodes mounted to the housing 184. The electrodes may include an RV tip electrode 186 at the distal end 182, an RV ring electrode 188, a coil electrode, and/or the like. The RV ring electrode 188 or another electrode may be located at or proximate to a proximal end 190 of the IMD 180, opposite the RV tip electrode 186.

The RV tip electrode 186 may represent the RV near field electrode 108 shown in Figure 2. The RV ring electrode 188 and the housing 184 (i.e., the can electrode) may represent the far field electrodes 110. In an example, the IMD 180 may include additional electrodes, such as a defibrillation coil. The bipolar atrial sensing vector may be defined by the RV tip electrode 186 and one of the RV ring electrode 188 or the housing 184.

Referring back to Figure 2, the IMD 100 may reconfigure the connection between the electrodes and the sensing circuitry 107 within the housing to establish the atrial sensing vector defined by one RV near field electrode 108 and one far field electrode 110. For example, the sensing circuitry 107 may include electrical terminals that are designed to receive inputs from atrial electrodes. The sensing circuitry 107 may be reconfigured so that the RV near field electrode 108 and the far field electrode 110 convey signals to the RA inputs. As such, the RA signal inputs to the RA terminals are replaced by signals collected by non-RA electrodes. The RV electrodes may be electrically connected to the RA terminals.

In a first example with reference to the embodiment shown in Figure 3, the RV tip electrode 136 and the RV coil electrode 140 define the atrial sensing vector. The sensing circuitry 107 may be reconfigured so that a first conductor 144 connected to the RV tip electrode 136 is electrically connected to a first RA terminal on the housing 132, and a second conductor 144 connected to the RV coil electrode 140 may be electrically connected to a second RA terminal on the housing 132. At least one of the first and second RA terminals may be designed for receiving input from an RA tip electrode or an RA ring electrode, neither of which is present.

In a first example, these RV electrodes may be connected to the RA terminals via internal modification to the electronic circuitry within the housing 132. For example, an electrical circuit pathway along the sensing circuitry 107 that extends from an RV tip terminal may be electrically connected (e.g., shorted) to a parallel circuit pathway that extends from an RA tip terminal. When an electrical sensing signal is received at the RV tip terminal from the RV tip electrode, the sensing signal may be conveyed along both circuit pathways to represent both the RA tip signal and the RV tip signal.

In a second example, the RV electrodes may be connected to the RA terminals via modification of the lead 134. For example, the conductor 144 connected to one of the RV electrodes may split into two branches at or near the proximal end of the lead 134. The two branches may be electrically connected to two different input terminals on the housing 132. Signals collected by the RV electrode may then be conveyed to both of the corresponding RV terminal and the corresponding RA terminal. For example, the conductor 144 connected to the RV tip electrode 136 may split into two branches that connect to two different connectors that are input into the RA tip terminal and the RV tip terminal, respectively.

In a third example, an external adaptor device may be installed to avoid modifying the lead 134 or the internal sensing circuitry 107 within the housing 132. The adapter device may be connected between the proximal end of the lead 134 and the housing 132. The adapter device may establish electrical connections between the conductors 144 of the lead 134 and corresponding terminals of the housing 132. Some example input configurations are shown in Table 1 below. These input configurations can be for single vector VDD mode sensing and/or pacing operations.

| ICD/CRT-D Sense/Pace Input | Lead electrode coupled to input |
|---|---|
| RA tip | RV near field (e.g. RV tip, ring) or far field electrode (e.g. can) |
| RA ring | Far field electrode (e.g. RV coil, can) |
| RV tip | RV near field (e.g. RV tip) electrode |
| RV ring | RV near field (e.g. RV ring) or far field electrode (e.g. RV coil) |

With reference to Figure 2, the RV near field electrode 108 that is selected for the atrial sensing vector may be electrically connected to a first RA terminal of the IMD 100 and a first RV terminal of the IMD 100. The far field electrode 110 that is selected for the atrial sensing vector may be electrically connected to a second RA terminal of the IMD 100 and a second RV terminal of the IMD 100. The atrial sensing vector can be defined by a multitude of different electrode pairs. The controller 102 may test different candidate atrial sensing vectors to select one atrial sensing vector that has desirable properties (e.g., sensitivity, reliability, signal quality, and/or the like).

In an embodiment, the IMD 100 may perform a method of collecting atrial sensing data of a patient's heart. The method may be performed using an IMD free of any atrial electrodes, whether on the housing of the IMD or a lead connected to the housing. The method may be performed using the IMD 100 shown in Figure 2, and optionally one of the example applications and embodiments shown in Figures 3 through 6. At a first step, an atrial sensing vector is defined via sensing circuitry 107 of the IMD 100. The atrial sensing vector includes an RV near field electrode 108 of the IMD and a far field electrode 110 of the IMD. The RV near field electrode 108 may be located (e.g., implanted) within the RV of the heart of the patient and either in contact with ventricular myocardial tissue of the heart or within a threshold proximity of the ventricular myocardial tissue. The far field electrode 110 may be positioned beyond the threshold proximity of the ventricular myocardial tissue. The atrial sensing data may be associated with atrial cardiac activity.

At a second step, the electrodes 108, 110 that define the atrial sensing vector may collect atrial sensing data of the heart. At a third step, the controller 102 (e.g., the one or more processors 104) may receive the atrial sensing data that is collected via the atrial sensing vector. The atrial sensing data may be communicated to the controller 102 from the sensing circuitry 107 that is coupled to the electrodes 108, 110.

At a fourth step, the controller 102 may analyze the atrial sensing data. At a fifth step, in response to the controller 102 determining that stimulation therapy is appropriate based on the analysis of the atrial sensing data, the controller 102 may perform the stimulation therapy on the heart. In an example, the stimulation therapy may be VDD mode pacing.

The sensing circuitry 107 may define a ventricular sensing vector to collect ventricular sensing data. The ventricular sensing data may be associated with RV cardiac activity. In various examples, the ventricular sensing vector may be different from the atrial sensing vector, although one of the electrodes may be used to define both vectors. In an example, the sensing circuitry 107 may define the ventricular sensing vector between the RV near field electrode 108 that is used to define the atrial sensing vector and a third electrode of the IMD that is coupled to the sensing circuitry 108. Example ventricular sensing vectors can include a first vector between the RV tip electrode and the RV coil electrode, a second vector between the RV tip electrode and the RV ring electrode, a third vector between the RV ring electrode and the RV coil electrode, a fourth vector between the RV tip electrode and the can electrode, and a fifth electrode between the RV ring electrode and the can electrode. In another example, the ventricular sensing vector may use the same electrode pair as the atrial sensing vector. The electrode pair in an example is the RV tip electrode and the RV coil electrode. At least one different electrode can be used to define the electrode pair in other examples.

In an embodiment, the IMD 100 is designed to perform a method of selecting the atrial sensing vector during sinus rhythm. Selecting the atrial sensing vector means selecting the two electrodes that will define the bipolar atrial sensing vector and will be used to monitor atrial activity.

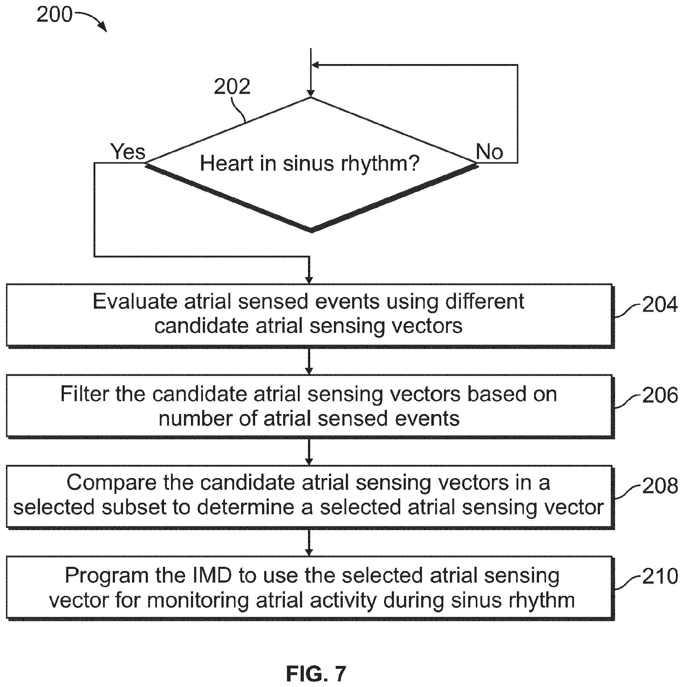

Figure 7 is a flow chart 200 of a method for selecting the atrial sensing vector for a sinus rhythm cardiac mode. The method may be performed in whole or in part by the one or more processors 104 (e.g., the controller 102) of the IMD 100. In different embodiments, the method may include different steps not shown in Figure 7, may omit one or more of the steps shown in Figure 7, and/or may have a different order of the steps than shown in Figure 7.

At step 202, the one or more processors 104 determine whether the intrinsic rhythm of the heart is sinus. Sinus rhythm may be a prerequisite for the method. If the heart is not in sinus rhythm, the IMD 100 may stop performing the method. The IMD 100 may check whether the intrinsic rhythm is sinus again after a designated period of time. If the one or more processors 104 confirm that the heart is in sinus rhythm, the method continues to step 204. The one or more processors 104 may determine whether the intrinsic rhythm is sinus based on one or more factors. One factor may be a comparison of rates in each chamber. For example, if the difference in atrial rate vs. ventricular rate is less than 10 beats per minute (bpm), then the intrinsic rhythm may be sinus. A second factor may be a comparison of R-R intervals measured from the ventricular sensing channel. For example, if the R-R interval variability is less than 50 milliseconds (ms) and the R-R interval duration is greater than 600 ms, then the intrinsic rhythm may be sinus. A third factor may be a morphology of ventricular sensed events. For example, if a morphology matching score is at least a threshold percentile (e.g., 90%), then the intrinsic rhythm may be sinus. A fourth factor may be a comparison of P-R intervals measured from atrial and ventricular sensing channels. For example, if the P-R interval variability is less than 30 ms, then the intrinsic rhythm may be sinus.

At step 204, the one or more processors evaluate sensed cardiac events using multiple different candidate atrial sensing vectors. Each of the candidate atrial sensing vectors is an electrode pair that includes an RV near field electrode 108 and a far field electrode 110. In an example, the RV near field electrode 108 may be an RV tip electrode or an RV ring electrode. The far field electrode 110 may be a coil electrode or a can electrode on the housing. The coil electrode may be an RV coil, an SVC coil, or the like. The candidate atrial sensing vectors are defined by different combinations of the RV near field electrode and the far field electrode.

The one or more processors 104 may receive and analyze atrial sensing data collected by each of the candidate atrial sensing vectors. The different candidate atrial sensing vectors may be used in sequence to sense atrial activity. For example, a first candidate atrial sensing vector may sense atrial activity during a first time period, a second candidate atrial sensing vector may sense atrial activity during a second time period (after the first time period), and so on. The one or more processors 104 may also evaluate sensed cardiac events, such as ventricular sensed events, using at least one ventricular sensing vector.

At step 206, the one or more processors 104 may filter the candidate atrial sensing vectors based on the number of sensed cardiac events detected by the candidate atrial sensing vectors. For example, the one or more processors 104 may identify a subset of the candidate atrial sensing vectors that have no more than three sensed cardiac events per sensed cardiac event detected via the ventricular sensing vector. Stated differently, if a given candidate atrial sensing vector yields more than three times the number of sensed cardiac events than the number of sensed cardiac events detected via the ventricular sensing vector, that candidate atrial sensing vector is excluded from the subset (e.g., is not selected as one of the identified candidate sensing vectors).

At step 208, the one or more processors 104 compare the candidate atrial sensing vectors that are in the selected subset of candidate atrial sensing vectors to determine a selected atrial sensing vector. The one or more processors 104 may determine which of the atrial sensing vectors in the subset has the greatest sensitivity ratio and/or the lowest atrial refractory period. The sensitivity ratio may refer to a P-wave to T-wave amplitude ratio. The atrial refractory period may refer to the duration between P-wave detection and T-wave detection. The selected atrial sensing vector may be the vector in the subset that has the greatest sensitivity ratio and/or the lowest atrial refractory period.

At step 210, the one or more processors 104 may automatically program (e.g., reprogram) an atrial sensing algorithm of the IMD 100 to use the selected atrial sensing vector for monitoring atrial activity during sinus rhythm (e.g., the sinus cardiac mode). The one or more processors 104 may program the atrial sensing algorithm by modifying one or more sensing parameters. For example, an atrial refractory period sensing parameter may be selected as the time duration between P-wave detection and T-wave detection via the selected atrial sensing vector. In another example, a post ventricular atrial refractory period (PVARP) sensing parameter may be selected as the time duration between R-wave detection and T-wave detection via the selected atrial sensing vector. In another example, an atrial maximum sensitivity sensing parameter may be selected as the far field P-wave amplitude. Optionally, a positive-negative safety factor value may be applied to the values of some or all of the sensing parameters to provide margin. Optionally, the one or more processors 104 may modify at least one sensing parameter that is different from the sensing parameters described in the examples.

In an embodiment, the IMD 100 is designed to perform a separate but similar method of selecting the atrial sensing vector during a pacing mode of operation. For example, the atrial sensing vector that is selected for the pacing mode may be different than the atrial sensing vector that is selected for sinus rhythm.

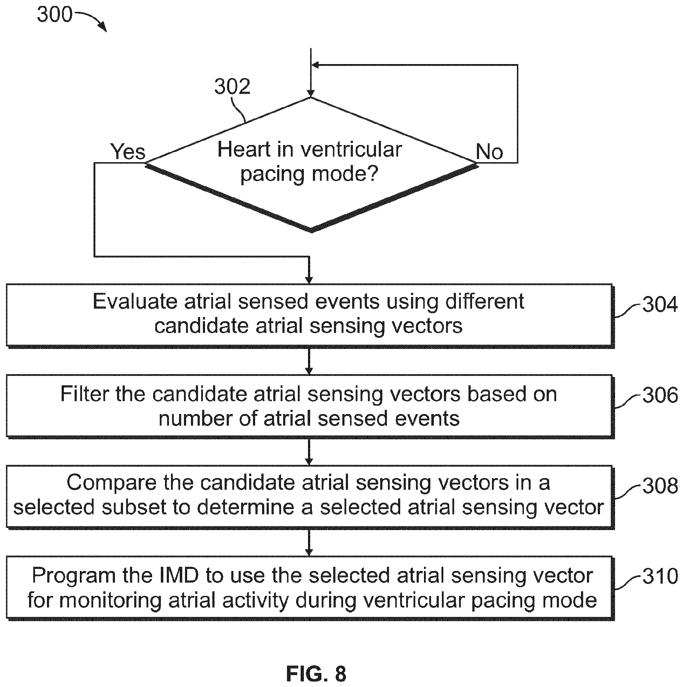

Figure 8 is a flow chart 300 of a method for selecting the atrial sensing vector for the pacing mode of operation. The method may be performed by the one or more processors 104 (e.g., the controller 102) of the IMD 100. The method may be similar to the method described above with reference to Figure 7. The atrial sensing vector that is selected during the method described in Figure 8 may be used to monitor atrial activity following ventricular pacing. In different embodiments, the method may include different steps not shown in Figure 8, may omit one or more of the steps shown in Figure 8, and/or may have a different order of the steps than shown in Figure 8.

At step 302, the one or more processors 104 determine whether the heart is in a ventricular pacing mode of operation. The ventricular pacing mode occurs immediately before and/or after a ventricular pacing pulse is delivered by the IMD 100 to the ventricular myocardium.

At step 304, the one or more processors 104 evaluate sensed cardiac events using multiple different candidate atrial sensing vectors. As described above with reference to the method in Figure 7, each of the candidate atrial sensing vectors is an electrode pair that includes an RV near field electrode 108 and a far field electrode 110. In each candidate sensing vector, the RV near field electrode 108 may be an RV tip electrode or an RV ring electrode. The far field electrode 110 may be a coil electrode or the can electrode on the housing. The coil electrode may be the RV coil, the SVC coil, or the like. The one or more processors 104 may receive and analyze atrial sensing data collected by each of the candidate atrial sensing vectors immediately before and/or after a ventricular pacing pulse is delivered by the IMD to the ventricular myocardium. The different candidate atrial sensing vectors may be used in sequence to sense atrial activity. For example, a first candidate atrial sensing vector may sense atrial activity immediately before and/or after a first ventricular pacing pulse or a first series of ventricular pacing pulses, a second candidate atrial sensing vector may sense atrial activity immediately before and/or after a second ventricular pacing pulse or a second series of ventricular pacing pulses, and so on. The one or more processors 104 may also evaluate sensed cardiac events using at least one ventricular sensing vector.

At step 306, the one or more processors 104 may filter the candidate atrial sensing vectors based on the number of sensed cardiac events detected by the candidate atrial sensing vectors. For example, the one or more processors 104 may identify a subset of the candidate atrial sensing vectors that have no more than three sensed cardiac events per sensed cardiac event detected via the ventricular sensing vector. Stated differently, if a given candidate atrial sensing vector yields more than three times the number of sensed cardiac events than the number detected by the ventricular sensing vector, that candidate atrial sensing vector is excluded from the subset (e.g., is not selected as one of the identified candidate sensing vectors).

At step 308, the one or more processors 104 may compare the candidate atrial sensing vectors that are in the selected subset of candidate atrial sensing vectors to determine a selected atrial sensing vector for the pacing mode of operation. The one or more processors 104 may determine which of the atrial sensing vectors in the subset has the greatest sensitivity ratio and/or the lowest (e.g., shortest) atrial refractory period. The sensitivity ratio refers to the P-wave to T-wave amplitude ratio. The atrial refractory period refers to the duration between P-wave detection and T-wave detection. The selected atrial sensing vector may be the vector in the subset that has the greatest sensitivity ratio and/or the lowest atrial refractory period.

At step 310, the one or more processors 104 may automatically program (e.g., reprogram) an atrial sensing algorithm of the IMD to use the selected atrial sensing vector for monitoring atrial activity during the ventricular pacing mode of operation. The one or more processors 104 may program the atrial sensing algorithm by modifying one or more sensing parameters. For example, the PVARP sensing parameter may be set equal to the time duration between pacing pulse delivery and T-wave detection. A positive-negative safety factor value may be applied to the values of some or all of the sensing parameters to provide margin.

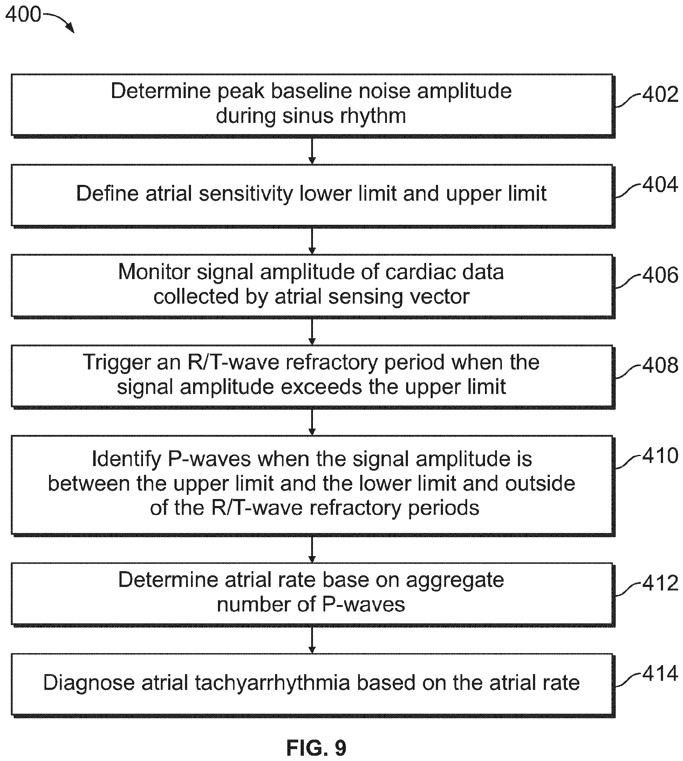

Figure 9 is a flow chart 400 of a method of detecting P-waves in cardiac data. The P-waves that are detected may be used to diagnose atrial tachyarrhythmias. The method may be performed by the IMD 100 described herein, including the one or more processors 104 (e.g., controller 102) thereof. The method may be performed without using any dedicated atrial sensing electrodes. Conventionally, detection of P-waves and diagnosis of atrial tachyarrhythmias is a unique challenge for a VDD mode system without any dedicated atrial sensing electrodes given the arrhythmia's cycle length is often less than the individual's QT interval. The IMD 100 may be designed to perform this method of detecting P-waves as well as the methods of selecting the atrial sensing vectors during different cardiac modes (e.g., sinus rhythm, ventricular pacing, etc.). The method of detecting P-waves may be a discrete, separate process from selecting atrial sensing vectors and/or modifying atrial sensing parameters. In different embodiments, the method may include different steps not shown in Figure 9, may omit one or more of the steps shown in Figure 9, and/or may have a different order of the steps than shown in Figure 9.

At step 402, the one or more processors 104 may determine a peak baseline noise amplitude (in mV) during sinus rhythm. The peak baseline noise amplitude may be determined by evaluating amplitude measured on the atrial sensing vector (e.g., channel) between sequential T-wave and P-wave detections. At step 404, the one or more processors 104 may define an atrial sensitivity upper limit and an atrial sensitivity lower limit (both in mV). In an example, the value of the upper limit may be selected as the maximum P-wave amplitude during sinus rhythm. The upper limit value optionally may incorporate a safety margin. In an example, the value of the lower limit may be selected as the peak baseline noise amplitude determined at step 402, optionally incorporating a safety margin.

At step 406, the absolute signal amplitude of the atrial sensing data collected by the atrial sensing vector is monitored over time in reference to the atrial sensitivity upper and lower limits.

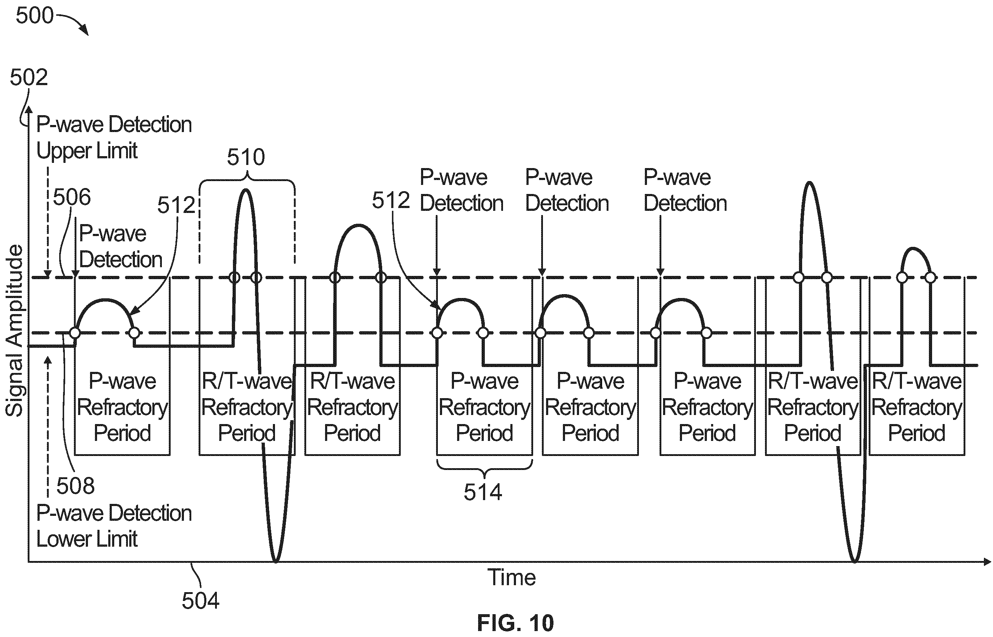

Figure 10 is a graph 500 depicting atrial signal amplitude 502 over time 504. The atrial signal amplitude may be collected by the bipolar atrial sensing vector of the IMD 100 that includes the RV near field electrode 108 and a far field electrode 110, without using any dedicated RA electrodes. The cardiac data may be electrocardiogram data collected by the IMD 100 when the IMD 100 is implanted in the patient. The graph shows the atrial sensitivity upper limit 506 and the atrial sensitivity lower limit 508.

In an example, the method is designed to avoid or at least reduce the occurrence of R/T-wave oversensing on the atrial sensing vector of the IMD 100. For example, in response to detecting an absolute signal amplitude that exceeds the atrial sensitivity upper limit, the one or more processors 104 trigger a sensing refractory period. The one or more processors 104 do not count any potential P-waves that occur within the refractory period.

At step 408, the one or more processors 104 trigger an R/T-wave refractory period 510 each time the absolute signal amplitude exceeds the atrial sensitivity upper limit 506. The R/T-wave refractory period 510 is a blanking window that extends both before and after the time that the absolute signal amplitude exceeds the upper limit. The one or more processors 104 do not detect (e.g., identify) any P-waves that occur during the R/T-wave refractory period. The duration (or width) of the R/T-wave refractory period 510 may be based on the QRS/T-wave duration. Defining the duration of the R/T-wave refractory period 510 based on the width of the QRS/T-wave complex may beneficially customize the sensing parameter set based on the specific patient. In an example, the value of the R/T-wave refractory period 510 may be calculated by the one or more processors 104 as +/- half of the QRS/T-wave width, optionally incorporating a safety margin. For example, the value of the R/T-wave refractory period 510 may be the QRS/T-wave refractory period divided by two, and may extend in both positive and negative directions from the time that the absolute signal amplitude crosses the upper limit.

At step 410, the one or more processors 104 identify P-waves 512 in the signal over time. In an example, the one or more processors 104 identify a P-wave if certain conditions are satisfied. One condition may be that the signal recorded on the atrial sensing channel has an amplitude that exceeds the lower limit 508 but not the upper limit 510. For example, the absolute signal amplitude may cross the lower limit 508 to be between the upper and lower limits 506, 508 at a first time, and may cross the lower limit 508 again at a second time without crossing the upper limit 506 between the first and second times. A second condition of identifying P-waves may be that the P-wave does not overlap in time with an R/T-wave refractory period 510. For example, if the time period between the first and second times overlaps with an identified R/T-wave refractory period 510 at step 408, then no P-wave is detected during that time period. In conclusion, the one or more processors 104 may identify a P-wave 512 in an example if the absolute signal amplitude exceeds the lower limit 508 without exceeding the upper limit 506 before crossing the lower limit 508 a second time, and if that time period is discrete from an R/T-wave refractory period 510. Stated differently, the one or more processors 104 may label a first signal event as an R/T wave (e.g., R wave or T wave) in response to the absolute signal amplitude exceeding the atrial sensitivity upper limit 506. The one or more processors 104 may label a second signal event as a P-wave 512 in response to the absolute signal amplitude exceeding the atrial sensitivity lower limit 508 without thereafter exceeding the upper limit 506, and as long as the time period during which the absolute signal amplitude is between the upper and lower limits 506, 508 does not overlap an R/T refractory period 510.

In an example, the one or more processors 104 may trigger a P-wave sensing refractory period 514 for each P-wave 512 that is identified. During this refractory period 514, the one or more processors do not identify any additional P-waves. Thus, the one or more processors may identify only one P-wave 512 per refractory period 514. The P-wave refractory period 514 may have a duration that is based on the P-wave duration. For example, the duration of the P-wave refractory period 514 may be selected to be equal to the P-wave duration (e.g., width), optionally incorporating a safety margin. The P-wave duration may be the time period from a first time that the absolute signal amplitude exceeds the lower limit 508 to the second time that the absolute signal amplitude falls below the lower limit 508.

The one or more processors 104 may still monitor for and detect R/T waves during the P-wave refractory period 514. For example, as described above with reference to step 408, the one or more processors 104 may identify an R/T wave in response to the absolute signal amplitude exceeding the atrial sensitivity upper limit 506. Identification of an R/T wave triggers the R/T-wave refractory period 510, and no P-wave is identified that occurs within that R/T-wave refractory period 510. For example, the one or more processors 104 do not count or label any signal marker as a P-wave that occurs during an R/T-wave refractory period 510.

At step 412, the one or more processors 104 count or aggregate the number of identified P-waves 512 and use the number of P-waves 512 to determine (e.g., calculate) an atrial rate. The atrial rate may be beats per minute of the atrium. At step 414, the one or more processors 104 may use the atrial rate to diagnose an atrial tachyarrhythmia, such as by comparing the atrial rate to a designated threshold value. For example, if the atrial rate exceeds a designated threshold associated with atrial tachyarrhythmia, then the one or more processors 104 may determine that the patient is experiencing atrial tachyarrhythmia. The diagnosis of an atrial tachyarrhythmia may trigger the implantable pulse generator to switch pacing mode (e.g. VDD to VVI) with termination of the atrial arrythmia triggering reversion to the original pacing mode.

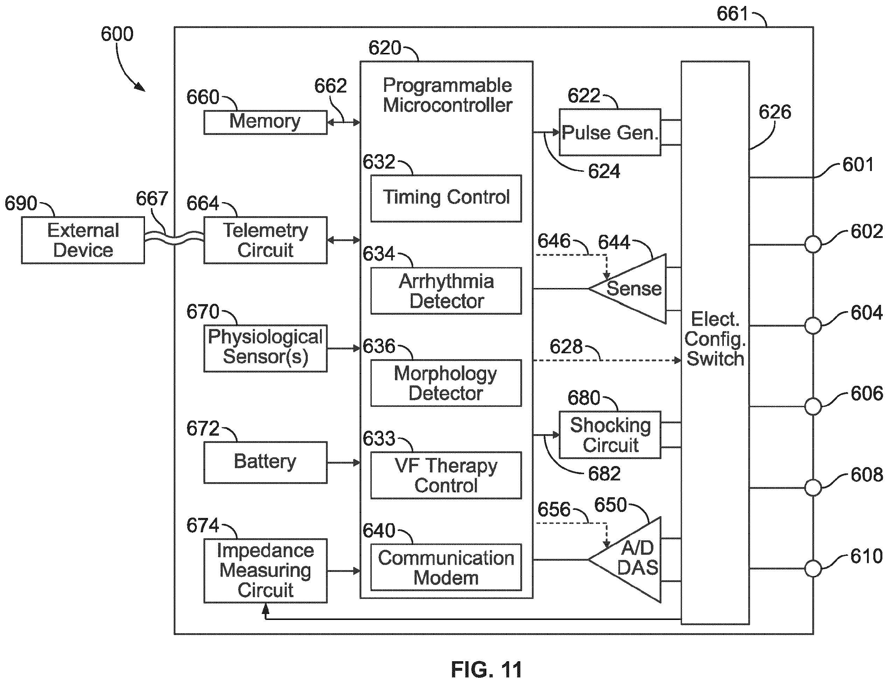

Figure 11 illustrates a block diagram of an exemplary IMD 600 that is configured to be implanted into the patient in accordance with embodiments herein. The IMD 600 may be represent any of the IMDs 50, 100, 130, 160, 180 described herein. The IMD 600 may treat both fast and slow arrhythmias with stimulation therapy, including cardioversion, pacing stimulation, an implantable cardioverter defibrillator, suspend tachycardia detection, tachyarrhythmia therapy, and/or the like.

The IMD 600 has a housing 661 to hold the electronic/computing components. The housing 661 (which is often referred to as the "can," "case," "encasing," or "can electrode") may be programmably selected to act as the return electrode for certain stimulus modes. The housing 661 further includes a connector (not shown) with a plurality of terminals 601, 602, 604, 606, 608, and 610. The terminals may be connected to a lead located within the heart. The lead has electrodes.

The IMD 600 includes a programmable microcontroller620 that controls various operations of the IMD 600, including cardiac monitoring and stimulation therapy. The microcontroller 620 may represent the controller 102 shown in Figure 2. The microcontroller 620 may include a microprocessor (or equivalent control circuitry), one or more processors, RAM and/or ROM memory, logic and timing circuitry, state machine circuitry, and I/O circuitry. The IMD 600 further includes a pulse generator 622 that generates stimulation pulses for connecting the desired electrodes to the appropriate I/O circuits, thereby facilitating electrode programmability. The pulse generator 622 may represent the pulse generator 112 in Figure 2. The IMD 600 may have an electrode configuration switch 626 that establishes electrical communication pathways with different selected combinations of the terminals for defining sensing vectors and pacing vectors. The switch 626 is controlled by control signals 628 from the microcontroller 620.

Optionally, the IMD 600 may include multiple pulse generators, similar to the pulse generator 622, where each pulse generator is coupled to one or more leads/electrodes and controlled by the microcontroller 620 to deliver select stimulus pulse(s) to the corresponding one or more electrodes. The IMD 600 includes sensing circuit 644 selectively coupled to one or more electrodes that perform sensing operations, through the switch 626 to detect the presence of cardiac activity in the chamber of the heart. The output of the sensing circuit 644 is connected to the microcontroller 620 which, in turn, triggers, or inhibits the pulse generator 622 in response to the absence or presence of cardiac activity. The sensing circuit 644 receives a control signal 646 from the microcontroller 620 for purposes of controlling the gain, threshold, polarization charge removal circuitry (not shown), and the timing of any blocking circuitry (not shown) coupled to the inputs of the sensing circuit 624.

In the example of Figure 11, one sensing circuit 644 is illustrated. Optionally, the IMD 600 may include multiple sensing circuits 644, where each sensing circuit is coupled to one or more leads/electrodes and controlled by the microcontroller 620 to sense electrical activity detected at the corresponding one or more electrodes. The sensing circuit 624 may operate in, for example, a unipolar sensing configuration or a bipolar sensing configuration.

The IMD 600 further includes an analog-to-digital (A/D) data acquisition system (DAS) 650 coupled to one or more electrodes via the switch 626 to sample cardiac signals across any pair of desired electrodes. The A/D converter 650 is configured to acquire intracardiac electrogram signals, convert the raw analog data into digital data and store the digital data for later processing and/or telemetric transmission to an external device 690 (e.g., a programmer, local transceiver, or a diagnostic system analyzer). The A/D converter 650 is controlled by a control signal 656 from the microcontroller 620.

The microcontroller 620 is operably coupled to a memory 660 by a suitable data/address bus 662. The programmable operating parameters used by the microcontroller 620 are stored in the memory 660 and used to customize the operation of the IMD 600 to suit the needs of a particular patient. The operating parameters of the IMD 600 may be non-invasively programmed into the memory 660 through a telemetry circuit 664 in telemetric communication via communication link 667 (e.g., MICS, Bluetooth low energy, and/or the like) with the external device 690.

The IMD 600 can further include one or more physiological sensors 670. Such sensors are commonly referred to as "rate-responsive" sensors because they are typically used to adjust pacing stimulation rates according to the exercise state of the patient. However, the physiological sensor 670 may further be used to detect changes in cardiac output, changes in the physiological condition of the heart, or diurnal changes in activity (e.g., detecting sleep and wake states). Signals generated by the physiological sensors 670 are passed to the microcontroller 620 for analysis. While shown as being included within the IMD 600, the physiological sensor(s) 670 may be external to the IMD 600, yet still, be implanted within or carried by the patient. Examples of physiological sensors might include sensors that, for example, sense respiration rate, pH of blood, ventricular gradient, activity, position/posture, minute ventilation, and/or the like.

A battery 672 provides operating power to all of the components in the IMD 600. The battery 672 is capable of operating at low current drains for extended periods of time, and is capable of providing a high-current pulses (for capacitor charging) when the patient requires a shock pulse (e.g., in excess of 2 A, at voltages above 2 V, for periods of 10 seconds or more). The battery 672 also desirably has a predictable discharge characteristic so that elective replacement time can be detected. As one example, the IMD 600 employs lithium/silver vanadium oxide batteries.

The IMD 600 further includes an impedance measuring circuit 674, which can be used for many things, including sensing respiration phase. The impedance measuring circuit 674 is coupled to the switch 626 so that any desired electrode and/or terminal may be used to measure impedance in connection with monitoring respiration phase. The IMD 600 is further equipped with a communication modem (modulator/demodulator) 640 to enable wireless communication with other devices, implanted devices and/or external devices. In one implementation, the communication modem 640 may use high frequency modulation of a signal transmitted between a pair of electrodes. As one example, the signals may be transmitted in a high frequency range of approximately 10-80 kHz, as such signals travel through the body tissue and fluids without stimulating the heart or being felt by the patient.

The IMD 600 in an example includes a shocking circuit 680 that generates defibrillation shocking pulses (e.g., shocks). The shocking circuit 680 may be the shocking circuit 114 shown in Figure 2. The shocking circuit 680 may be controlled by the microcontroller 620 by a control signal 682. The microcontroller 620 may control the shocking circuit 680 by way of a timing control module 632. The timing control module 632 is used to control various timing parameters, such as stimulation pulses (e.g., pacing rate, atria-ventricular (AV) delay, atrial interconduction (A-A) delay, ventricular interconduction (V-V) delay, etc.) as well as to keep track of the timing of RR-intervals, refractory periods, blanking intervals, noise detection windows, evoked response windows, alert intervals, marker channel timing, and the like.

Although not shown, the microcontroller 620 may further include other dedicated circuitry and/or firmware/software components that assist in monitoring various conditions of the patient's heart and managing pacing therapies. The microcontroller 620 further includes an arrhythmia detector 634, a morphology detector 636 and multi-phase therapy controller 633. The arrhythmia detector 634 may be configured to apply one or more arrhythmia detection algorithms for detecting arrhythmia conditions. By way of example, the arrhythmia detector 634 may apply various detection algorithms. The arrhythmia detector 634 may be configured to declare a ventricular fibrillation episode based on the cardiac events.

The morphology detector 636 is configured to review and analyze one or more features of the morphology of cardiac activity signals. For example, in accordance with embodiments herein, the morphology detector 636 may analyze the morphology of detected R waves, where such morphology is then utilized to determine whether to include or exclude one or more beats from further analysis. For example, the morphology detector 636 may be utilized to identify non-conducted ventricular events, such as ventricular fibrillation and the like.

The multi-phase therapy controller 633 may identify a multi-phase therapy based on a ventricular fibrillation episode. The multi-phase therapy may include a pacing therapy. The therapy controller 633 may manage delivery of the burst pacing therapy at a pacing site in a coordinated manner after the one or more shocks. The pacing site may be located at a target SOI, such as a His Bundle. Optionally, other pacing sites may be located at one of a left ventricular (LV) site or a right ventricular (RV) site. The therapy controller 633 may manage delivery of the shock along a shocking vector between shocking electrodes.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

The term "approximately" as used herein includes a threshold range of values above and below the stated value. For example, the threshold range may be +/- 5%, 3%, 2%, or the like.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the inventive subject matter without departing from its scope. While the dimensions and types of materials described herein are intended to define the parameters of the inventive subject matter, they are by no means limiting and are exemplary embodiments. Many other embodiments will be apparent to one of ordinary skill in the art upon reviewing the above description. The scope of the inventive subject matter should, therefore, be determined with reference to the appended claims**.** In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. An implantable medical device (100, 130, 180, 600) comprising:
a housing (132, 184, 661) that contains one or more processors (104, 620); and
a right ventricle (RV) near field (NF) electrode (108) electrically connected to the one or more processors (104, 620), the RV NF electrode (108) configured to be located within the RV of a heart (10) of a patient and either in contact with ventricular myocardial tissue of the heart (10) or within a threshold proximity of the ventricular myocardial tissue;
a far field (FF) electrode (110) configured to be positioned beyond the threshold proximity of the ventricular myocardial tissue;
a lead (134) having the RV NF electrode (108) and the FF electrode (110); and
sensing circuitry (107, 644) coupled to the RV NF electrode (108) and the FF electrode (110) and configured to define an atrial sensing vector between the RV NF electrode (108) and the FF electrode (110) to collect atrial sensing data associated with atrial cardiac activity,
wherein the one or more processors (104, 620) are configured to receive the atrial sensing data and are configured to perform VDD mode pacing on the heart (10) based on the atrial sensing data.

2. The implantable medical device of claim 1, further comprising a third electrode, the sensing circuitry (107, 644) coupled to the third electrode and configured to define a ventricular sensing vector between the third electrode and the RV NF electrode (108) and configured to collect ventricular sensing data.

3. The implantable medical device of claim 1 or 2, wherein the RV NF electrode (108) is one of an RV tip electrode (136) or an RV ring electrode (138).

4. The implantable medical device of any one of claims 1 to 3, wherein the sensing circuitry (107, 644) is configured to establish a conductive pathway from the RV NF electrode (108) to both an RV terminal and right atrium terminal on or within the housing (132, 184, 661).

5. The implantable medical device of any one of claims 1 to 4, wherein the RV NF electrode (108) is located proximate to a distal end (142) of the lead (134), and the FF electrode (110) is spaced apart from the RV NF electrode (108) by at least 1 cm.

6. The implantable medical device of claim 5, wherein the FF electrode (110) is a coil electrode (140) configured to be located near or partially within a right atrium of the heart.

7. The implantable medical device of claim 5 or 6, wherein the lead (134) is the only lead of the implantable medical device (100, 130, 600).

8. The implantable medical device of any one of claims 5 to 7, wherein the RV NF electrode (108) is electrically connected to a first conductor (144) that extends along a length of the lead (134) to a proximal end of the lead (134), wherein the first conductor (144) is electrically connected to both a first RV terminal of the housing (132, 661) and a first right atrium (RA) terminal of the housing (132, 661) so that signals collected by the RV NF electrode (108) are conveyed to both the first RV terminal and the first RA terminal.

9. The implantable medical device of claim 8, wherein the FF electrode (110) is a coil electrode (140) and is electrically connected to a second conductor (144) that extends along a length of the lead (134) to the proximal end of the lead (134), wherein the second conductor (144) is electrically connected to both a second RV terminal of the housing (132, 661) and a second RA terminal of the housing (132, 661) so that signals collected by the coil electrode are conveyed to both the second RV terminal and the second RA terminal.

10. The implantable medical device of any one of claims 1 to 9, wherein the implantable medical device (100, 130, 180, 600) is free of any atrial electrodes.

11. The implantable medical device of any one of claims 1 to 10, wherein the implantable medical device (100, 130, 600) is an implantable cardioverter-defibrillation (ICD) device or a cardiac resynchronization therapy-defibrillation (CRT-D) device.

12. The implantable medical device of any one of claims 1 to 11, wherein the one or more processors (104, 620) are configured to analyze the atrial sensing data collected via the atrial sensing vector to detect P-waves over time and determine an atrial rate based on the P-waves that are detected.

13. The implantable medical device of claim 12, wherein the one or more processors (104, 620) are configured to detect the P-waves by:
defining an atrial sensitivity upper limit and an atrial sensitivity lower limit; monitoring an absolute signal amplitude of the atrial sensing data over time; triggering R/T-wave refractory periods responsive to the absolute signal amplitude exceeding the atrial sensitivity upper limit; and
identifying the P-waves at times that the absolute signal amplitude is between the atrial sensitivity upper and lower limits and is outside of the R/T-wave refractory periods.

14. The implantable medical device of claim 12 or 13, wherein the one or more processors (104, 620) are configured to diagnose atrial tachyarrhythmia based on the atrial rate.

## Patentansprüche

1. Implantierbare medizinische Vorrichtung (100, 130, 180, 600), umfassend:
ein Gehäuse (132, 184, 661), das einen oder mehrere Prozessoren (104, 620) enthält; und
eine Nahfeldelektrode (NF-Elektrode) (108) für den rechten Ventrikel (RV), die elektrisch mit dem einen oder den mehreren Prozessoren (104, 620) verbunden ist, wobei die RV-NF-Elektrode (108) dazu konfiguriert ist, sich innerhalb des RV eines Herzens (10) eines Patienten zu befinden und entweder in Kontakt mit ventrikulärem Myokardgewebe des Herzens (10) zu stehen oder innerhalb einer Schwellennähe des ventrikulären Myokardgewebes zu sein;
eine Fernfeldelektrode (FF-Elektrode) (110), die dazu konfiguriert ist, jenseits der Schwellennähe des ventrikulären Myokardgewebes positioniert zu sein;
eine Leitung (134), die die RV-NF-Elektrode (108) und die FF-Elektrode (110) aufweist; und
eine Erfassungsschaltung (107, 644), die an die RV-NF-Elektrode (108) und die FF-Elektrode (110) gekoppelt und dazu konfiguriert ist, einen atrialen Erfassungsvektor zwischen der RV-NF-Elektrode (108) und der FF-Elektrode (110) zu definieren, um atriale Erfassungsdaten zu sammeln, die einer atrialen Herzaktivität zugeordnet sind,
wobei der eine oder die mehreren Prozessoren (104, 620) dazu konfiguriert sind, die atrialen Erfassungsdaten zu empfangen, und dazu konfiguriert sind, eine VDD-Modus-Stimulation an dem Herzen (10) basierend auf den atrialen Erfassungsdaten durchzuführen.

2. Implantierbare medizinische Vorrichtung nach Anspruch 1, ferner umfassend eine dritte Elektrode, wobei die Erfassungsschaltung (107, 644) an die dritte Elektrode gekoppelt und dazu konfiguriert ist, einen ventrikulären Erfassungsvektor zwischen der dritten Elektrode und der RV-NF-Elektrode (108) zu definieren, und dazu konfiguriert ist, ventrikuläre Erfassungsdaten zu sammeln.

3. Implantierbare medizinische Vorrichtung nach Anspruch 1 oder 2, wobei die RV-NF-Elektrode (108) eines von einer RV-Spitzenelektrode (136) oder einer RV-Ringelektrode (138) ist.

4. Implantierbare medizinische Vorrichtung nach einem der Ansprüche 1 bis 3, wobei die Erfassungsschaltung (107, 644) dazu konfiguriert ist, einen leitfähigen Weg von der RV-NF-Elektrode (108) sowohl zu einem RV-Anschluss als auch einem rechten Vorhofanschluss an oder innerhalb des Gehäuses (132, 184, 661) herzustellen.

5. Implantierbare medizinische Vorrichtung nach einem der Ansprüche 1 bis 4, wobei sich die RV-NF-Elektrode (108) nahe einem distalen Ende (142) der Leitung (134) befindet und die FF-Elektrode (110) um mindestens 1 cm von der RV-NF-Elektrode (108) beabstandet ist.

6. Implantierbare medizinische Vorrichtung nach Anspruch 5, wobei die FF-Elektrode (110) eine Spulenelektrode (140) ist, die dazu konfiguriert ist, sich in der Nähe oder teilweise innerhalb eines rechten Vorhofs des Herzens zu befinden.

7. Implantierbare medizinische Vorrichtung nach Anspruch 5 oder 6, wobei die Leitung (134) die einzige Leitung der implantierbaren medizinischen Vorrichtung (100, 130, 600) ist.

8. Implantierbare medizinische Vorrichtung nach einem der Ansprüche 5 bis 7, wobei die RV-NF-Elektrode (108) elektrisch mit einem ersten Leiter (144) verbunden ist, der sich entlang einer Länge der Leitung (134) zu einem proximalen Ende der Leitung (134) erstreckt, wobei der erste Leiter (144) elektrisch sowohl mit einem ersten RV-Anschluss des Gehäuses (132, 661) als auch mit einem ersten rechten Vorhofanschluss (RA-Anschluss) des Gehäuses (132, 661) verbunden ist, sodass durch die RV-NF-Elektrode (108) gesammelte Signale sowohl zu dem ersten RV-Anschluss als auch zu dem ersten RA-Anschluss übertragen werden.

9. Implantierbare medizinische Vorrichtung nach Anspruch 8, wobei die FF-Elektrode (110) eine Spulenelektrode (140) ist und elektrisch mit einem zweiten Leiter (144) verbunden ist, der sich entlang einer Länge der Leitung (134) zu dem proximalen Ende der Leitung (134) erstreckt, wobei der zweite Leiter (144) elektrisch sowohl mit einem zweiten RV-Anschluss des Gehäuses (132, 661) als auch mit einem zweiten RA-Anschluss des Gehäuses (132, 661) verbunden ist, sodass durch die Spulenelektrode gesammelte Signale sowohl zu dem zweiten RV-Anschluss als auch zu dem zweiten RA-Anschluss übertragen werden.

10. Implantierbare medizinische Vorrichtung nach einem der Ansprüche 1 bis 9, wobei die implantierbare medizinische Vorrichtung (100, 130, 180, 600) frei von jeglichen atrialen Elektroden ist.

11. Implantierbare medizinische Vorrichtung nach einem der Ansprüche 1 bis 10, wobei die implantierbare medizinische Vorrichtung (100, 130, 600) eine implantierbare Cardioverter-Defibrillationsvorrichtung (ICD-Vorrichtung) oder eine Vorrichtung zur kardialen Resynchronisationstherapie-Defibrillation (CRT-D-Vorrichtung) ist.

12. Implantierbare medizinische Vorrichtung nach einem der Ansprüche 1 bis 11, wobei der eine oder die mehreren Prozessoren (104, 620) dazu konfiguriert sind, die über den atrialen Erfassungsvektor gesammelten atrialen Erfassungsdaten zu analysieren, um P-Wellen im Verlauf der Zeit zu detektieren und eine atriale Frequenz basierend auf den P-Wellen, die detektiert werden, zu bestimmen.

13. Implantierbare medizinische Vorrichtung nach Anspruch 12, wobei der eine oder die mehreren Prozessoren (104, 620) dazu konfiguriert sind, die P-Wellen durch Folgendes zu detektieren:
Definieren einer oberen atrialen Empfindlichkeitsgrenze und einer unteren atrialen Empfindlichkeitsgrenze;
Überwachen einer absoluten Signalamplitude der atrialen Erfassungsdaten im Verlauf der Zeit;
Auslösen von R/T-Wellen-Refraktärperioden als Reaktion darauf, dass die absolute Signalamplitude die obere atriale Empfindlichkeitsgrenze überschreitet; und
Identifizieren der P-Wellen zu Zeiten, zu denen die absolute Signalamplitude zwischen der oberen und der unteren atrialen Empfindlichkeitsgrenze liegt und außerhalb der R/T-Wellen-Refraktärperioden liegt.

14. Implantierbare medizinische Vorrichtung nach Anspruch 12 oder 13, wobei der eine oder die mehreren Prozessoren (104, 620) dazu konfiguriert sind, atriale Tachyarrhythmie basierend auf der atrialen Frequenz zu diagnostizieren.

## Revendications

1. Dispositif médical implantable (100, 130, 180, 600) comprenant :
un boîtier (132, 184, 661) qui contient un ou plusieurs processeurs (104, 620) ; et
une électrode de champ proche (NF) de ventricule droit (RV) (108) connectée électriquement à l'un ou plusieurs processeurs (104, 620), l'électrode NF de RV (108) étant configurée pour être située à l'intérieur du RV d'un cœur (10) d'un patient et soit en contact avec un tissu myocardique ventriculaire du cœur (10) soit dans les limites d'une proximité seuil du tissu myocardique ventriculaire ;
une électrode de champ lointain (FF) (110) configurée pour être positionnée au-delà de la proximité seuil du tissu myocardique ventriculaire ;
une dérivation (134) ayant l'électrode NF de RV (108) et l'électrode FF (110) ; et
une circuiterie de détection (107, 644) couplée à l'électrode NF de RV (108) et à l'électrode FF (110) et configurée pour la définition d'un vecteur de détection auriculaire entre l'électrode NF de RV (108) et l'électrode FF (110) pour la collecte de données de détection auriculaire associées à une activité cardiaque auriculaire,
dans lequel l'un ou plusieurs processeurs (104, 620) sont configurés pour la réception des données de détection auriculaire et sont configurés pour la réalisation d'une stimulation en mode VDD sur le cœur (10) sur la base des données de détection auriculaire.

2. Dispositif médical implantable selon la revendication 1, comprenant en outre une troisième électrode, la circuiterie de détection (107, 644) étant couplée à la troisième électrode et configurée pour la définition d'un vecteur de détection ventriculaire entre la troisième électrode et l'électrode NF de RV (108) et configurée pour la collecte de données de détection ventriculaire.

3. Dispositif médical implantable selon la revendication 1 ou 2, dans lequel l'électrode NF de RV (108) est une parmi une électrode de pointe de RV (136) ou une électrode annulaire de RV (138).

4. Dispositif médical implantable selon l'une quelconque des revendications 1 à 3, dans lequel la circuiterie de détection (107, 644) est configurée pour l'établissement d'une voie conductrice de l'électrode NF de RV (108) à la fois à une borne de RV et à une borne d'oreillette droite sur le boîtier (132, 184, 661) ou à l'intérieur de celui-ci.

5. Dispositif médical implantable selon l'une quelconque des revendications 1 à 4, dans lequel l'électrode NF de RV (108) est située à proximité d'une extrémité distale (142) de la dérivation (134), et l'électrode FF (110) est espacée de l'électrode NF de RV (108) d'au moins 1 cm.

6. Dispositif médical implantable selon la revendication 5, dans lequel l'électrode FF (110) est une électrode hélicoïdale (140) configurée pour être située près d'une oreillette droite du cœur ou partiellement à l'intérieur de celle-ci.

7. Dispositif médical implantable selon la revendication 5 ou 6, dans lequel la dérivation (134) est la seule dérivation du dispositif médical implantable (100, 130, 600).

8. Dispositif médical implantable selon l'une quelconque des revendications 5 à 7, dans lequel l'électrode NF de RV (108) est connectée électriquement à un premier conducteur (144) qui s'étend le long d'une longueur de la dérivation (134) vers une extrémité proximale de la dérivation (134), dans lequel le premier conducteur (144) est connecté électriquement à la fois à une première borne de RV du boîtier (132, 661) et à une première borne d'oreillette droite (RA) du boîtier (132, 661) de sorte que des signaux collectés par l'électrode NF de RV (108) sont acheminés à la fois vers la première borne de RV et la première borne de RA.

9. Dispositif médical implantable selon la revendication 8, dans lequel l'électrode FF (110) est une électrode hélicoïdale (140) et est connectée électriquement à un second conducteur (144) qui s'étend le long d'une longueur de la dérivation (134) vers l'extrémité proximale de la dérivation (134), dans lequel le second conducteur (144) est connecté électriquement à la fois à une seconde borne de RV du boîtier (132, 661) et à une seconde borne de RA du boîtier (132, 661) de sorte que des signaux collectés par l'électrode hélicoïdale sont acheminés à la fois vers la seconde borne de RV et la seconde borne de RA.

10. Dispositif médical implantable selon l'une quelconque des revendications 1 à 9, dans lequel le dispositif médical implantable (100, 130, 180, 600) est exempt de toutes électrodes auriculaires.

11. Dispositif médical implantable selon l'une quelconque des revendications 1 à 10, dans lequel le dispositif médical implantable (100, 130, 600) est un dispositif de défibrillation automatique implantable (ICD) ou un dispositif de thérapie de resynchronisation cardiaque avec défibrillation (CRT-D).

12. Dispositif médical implantable selon l'une quelconque des revendications 1 à 11, dans lequel l'un ou plusieurs processeurs (104, 620) sont configurés pour l'analyse des données de détection auriculaire collectées via le vecteur de détection auriculaire pour la détection d'ondes P au fil du temps et la détermination d'une fréquence auriculaire sur la base des ondes P qui sont détectées.

13. Dispositif médical implantable selon la revendication 12, dans lequel l'un ou plusieurs processeurs (104, 620) sont configurés pour la détection des ondes P par :
la définition d'une limite supérieure de sensibilité auriculaire et d'une limite inférieure de sensibilité auriculaire ;
la surveillance d'une amplitude de signal absolue des données de détection auriculaire au fil du temps ;
le déclenchement de périodes réfractaires d'onde R/T en réponse à l'amplitude de signal absolue dépassant la limite supérieure de sensibilité auriculaire ; et
l'identification des ondes P à des instants où l'amplitude de signal absolue se situe entre les limites supérieure et inférieure de sensibilité auriculaire et est en dehors des périodes réfractaires d'onde R/T.

14. Dispositif médical implantable selon la revendication 12 ou 13, dans lequel l'un ou plusieurs processeurs (104, 620) sont configurés pour le diagnostic d'une tachyarythmie auriculaire sur la base de la fréquence auriculaire.
